# EUROPEAN PATENT APPLICATION

(11) **EP 2 700 709 A1**
(43) Date of publication of application: **26.02.2014**
(21) Application number: 12774375.5
(22) Date of filing: 20.04.2012
(51) Int. Cl.: C12N 5/10, C12N 5/071, C12N 5/0735

(54) **iPS CELL HAVING DIFFERENTIATION PROPENSITY FOR CORNEAL EPITHELIUM**

(30) Priority: 20.04.2011 JP 2011093846
(71) Applicant: Osaka University, Suita-shi Osaka 565-0871 (JP)
(72) Inventor: HAYASHI, Ryuhei, Suita-shi, Osaka 565-0871 (JP); NISHIDA, Kohji, Suita-shi, Osaka 565-0871 (JP)
(74) Representative: Sutcliffe, Nicholas Robert
(86) International application number: PCT/JP2012/060667
(87) International publication number: WO 2012/144582

(57) **Abstract**

A major object of the present invention is to provide a method for inducing cell differentiation into corneal epithelial stem cells and/or corneal epithelial cells, for the easy production of a corneal epithelial cell sheet having superior safety in view of the possibility of vascularization and the like occurring. A method for inducing differentiation of a pluripotent stem cell into a corneal epithelial stem cell and/or a corneal epithelial cell, the method comprising the step of culturing a pluripotent stem cell in the presence of a stromal cell or an amnion-derived factor.

## Description

### Technical Field

The present invention mainly relates to a method for inducing differentiation of pluripotent stem cells into corneal epithelial stem cells and/or corneal epithelial cells; a method for producing corneal epithelial stem cells and/or corneal epithelial cells; a corneal epithelial cell sheet obtained from the corneal epithelial stem cells; and induced pluripotent stem cells (iPS cell) used for the induction of cell differentiation into corneal epithelial stem cells and/or corneal epithelial cells.

### Background Art

Good visual function is extremely important for quality of life. Thus, society has a considerably high level of need for the development of a fundamental remedy for eye diseases that impair vision, in particular, refractory eye diseases. Known refractory eye diseases include corneal epithelial stem cell deficiency, such as Stevens-Johnson syndrome, ocular pemphigoid, and alkali injury. Corneal epithelial stem cell deficiency is caused by external injury or chronic inflammatory response due to autoimmunity or the like, and leads to irreversible dysfunction or loss of corneal epithelial stem cells in the corneal limbus, thereby causing opacified cornea. This results in loss of the original transparency of the cornea due to invasion of the conjunctival epithelium into the central cornea, thereby significantly decreasing visual function. As a method for recovering the vision of patients with a critical corneal disease, such as corneal epithelial stem cell deficiency, corneal transplantation has been performed. In particular, since it is not possible to obtain normal cornea from patients who have a bilateral eye disease, the only option for these patients is currently allograft cornea transplantation. However, since allograft cornea transplantation has problems including cornea donor shortage and rejection of the allograft cornea, there has been a need to develop a treatment using an autologous cell source.

For patients with a unilateral corneal disease, it is possible to obtain corneal epithelial stem cells from the peripheral region (corneal limbus) of the cornea of the normal eye. Hence, a method of transplanting a corneal sheet reproduced from these cells has been conducted (Non-Patent Literature 1). The corneal sheets reproduced from the corneal epithelial stem cells obtained from the patients themselves are autologous cells, and are thus free from rejection.

However, such transplantation cannot be conducted on patients who have a bilateral corneal disease, because the corneal epithelial stem cells cannot be obtained from them. In fact, many patients with corneal epithelial stem cell deficiency have bilateral symptoms. As a tissue regeneration technique for treating patients with a bilateral corneal disease by using autologous cells, a method for transplanting an autologous oral mucosal epithelial cell sheet produced from oral mucosal epithelial stem cells obtained from the same individual has been developed (Non-Patent Literature 2). This autologous oral mucosal epithelial cell sheet transplantation is already clinically applied with desirable outcomes. Specifically, among 15 cases, the cure rate of transparency is 93% (14/15 eyes) and the rate of vision improvement is 80% (12/15 eyes) throughout 1-year observation. As such, the autologous oral mucosal epithelial cell sheet transplantation has demonstrated successful recovery of visual function in eye disease patients.

On the other hand, from a long-term viewpoint, since oral mucosa has many blood vessels, patients who receive a transplant of an autologous oral mucosal epithelial cell sheet may eventually develop vascularization in the transplanted oral mucosal epithelial cell sheet. Vascularization may further cause a decrease in transparency of the oral mucosal epithelial cell sheet. For this reason, an ideal method is to reproduce the corneal epithelium itself from the autologous cells of patients. However, at present, no methods are available for reproducing cornea epithelium, in particular, a cornea epithelial sheet, from autologous cells of patients by inducing cell differentiation into corneal epithelial cells or corneal epithelial stem cells.

Currently, known methods for inducing cell differentiation into a specific cell can be applied only to a few specific cells. Examples include the SDIA method for inducing the differentiation of embryonic stem cells into neural stem cells, nerve cells, neural crest cells, or neural tubes (Patent Literature 2 and Non-Patent Literature 3). The characteristic of the SDIA method is the differentiation induction of embryonic stem cells in the presence of stromal cells. The method is considered to be a technique of suppressing the differentiation of embryonic stem cells into epidermal cells or the like, thereby inducing their differentiation into nerve cells, etc. More specifically, the SDIA method induces cell differentiation into nerve cells, etc., and is thus different from the technique of inducing cell differentiation into corneal epithelial cells or corneal epithelial stem cells, which are considered to be embryologically similar to epidermal cells.

The artificially induced pluripotent stem cells (iPS cells) recently developed by Yamanaka et al. can be established from readily obtainable somatic cells, such as fibroblasts, obtained from the same individual (Patent Literature 1). iPS cells are known for their superior pluripotency comparable to embryonic stem cells (ES cells). Regenerative medicine using iPS cells as a cell source is highly expected to be a means for simultaneously solving the problems of cornea donor shortage and allograft rejection. Further, use of iPS cells raises no ethical issues regarding destruction of the early embryo, because, unlike ES cells, it does not require a step of destroying an early embryo. However, at present, there have been no reports of successful inducing differentiation of human ES cells or human iPS cells into corneal epithelium.

### Citation List

### Patent Literature

Patent Literature 1: W02007/069666
Patent Literature 2: W02001/088100
Patent Literature 3: JP2004-261533
Patent Literature 4: JP2005-333904

### Non-Patent Literature

Non-Patent Literature 1: Nishida K., et al., Transplantation 2004, 77: 379-385.
Non-Patent Literature 2: Nishida K., et al., N Engl J Med 2004, 351: 1187-1195.
Non-Patent Literature 3: Kawasaki H., et al., Neuron 2000, 28: 31-40.

### Summary of Invention

### Technical Problem

An object of the present invention is to provide a method for producing corneal epithelial stem cells and/or corneal epithelial cells, and a method for inducing cell differentiation into corneal epithelial stem cells and/or corneal epithelial cells, for the production of a corneal epithelial cell sheet having superior safety in view of the possibility of vascularization and the like occurring, in a manner as easy as the production of known autologous oral mucosal epithelial cell sheets. Further, another object of the present invention is to provide corneal epithelial stem cells and/or a corneal epithelial cell sheet obtained from corneal epithelial cells, and a cell material used for induction of cell differentiation into corneal epithelial stem cells and/or corneal epithelial cells.

### Solution to Problem

The inventors of the present invention conducted extensive research to solve the above problems, and, surprisingly, found that it is possible to induce the differentiation of induced pluripotent stem cells into corneal epithelial cells or corneal epithelial stem cells by culturing the pluripotent stem cells in the presence of stromal cells. The inventors further found that it is possible to more efficiently induce cell differentiation into corneal epithelial stem cells and/or corneal epithelial cells by inducing the differentiation of induced pluripotent stem cells prepared from ocular surface epithelial cells. With further analysis based on these findings, the inventors completed the present invention.

Specifically, the present invention encompasses the inventions of the following aspects.
Item 1. A method for inducing differentiation of a pluripotent stem cell into a corneal epithelial stem cell and/or a corneal epithelial cell,
   the method comprising the step of culturing a pluripotent stem cell in the presence of a stromal cell or an amnion-derived factor.
Item 2. The method according to Item 1, wherein the pluripotent stem cell is cultured in a serum-free medium.
Item 3. The method according to Item 2, wherein the serum-free medium substantially does not contain BMP4 at least during the period from the beginning of the culture to 1 to 2 weeks after the culture.
Item 4. The method according to any one of Items 1 to 3, wherein the pluripotent stem cell is an embryonic stem cell (ES cell) or an induced pluripotent stem cell (iPS cell).
Item 5. The method according to Item 4, wherein the induced pluripotent stem cell is obtained by introducing a reprogramming factor, which can induce a cell into a pluripotent stem cell, into an ocular surface epithelium-derived cell.
Item 6. A method for producing a corneal epithelial stem cell and/or a corneal epithelial cell, comprising the step of:
   (1) culturing a pluripotent stem cell in the presence of a stromal cell or an amnion-derived factor; and
   (2) selecting a corneal epithelial stem cell and/or a corneal epithelial cell from among the cultured cells.
Item 7. The method according to Item 6, wherein the step of selecting a corneal epithelial stem cell and/or a corneal epithelial cell from among the cultured cells is a step of selecting a cell that has self-renewal ability and that expresses a corneal epithelial stem cell-specific marker.
Item 8. A corneal epithelial cell sheet originated from the corneal epithelial stem cell and/or the corneal epithelial cell obtained by the method according to Item 6 or 7.
Item 9. An induced pluripotent stem cell obtained by introducing a reprogramming factor, which can induce a cell into a pluripotent stem cell, into an ocular surface epithelium-derived cell.
Item 10. The induced pluripotent stem cell according to Item 9, wherein the induced pluripotent stem cell is used for differentiation induction into a corneal epithelial stem cell and/or a corneal epithelial cell.
Item 11. A cell material for inducing differentiation into a corneal epithelial stem cell and/or a corneal epithelial cell, the cell material comprising the induced pluripotent stem cell according to Item 9 or 10.

### Effects of Invention

The present invention provides, for the first time in the world, a method for producing corneal epithelial stem cells and/or corneal epithelial cells and a method for inducing cell differentiation into corneal epithelial stem cells and/or corneal epithelial cells from autologous cells, i.e., cells from the same individual. The corneal epithelial stem cells produced by the present invention, and a corneal epithelial cell sheet obtained from the corneal epithelial stem cells, can be made as easily as the production of oral mucosal epithelial cell sheets. Further, the corneal epithelial cells and corneal epithelial stem cells obtained by inducing the differentiation of cells removed from a patient, and the corneal epithelial cell sheet obtained from the corneal epithelial stem cells, will cause no rejection insofar as the cells or the sheet is returned to the same individual (autologous transplantation). In addition, the corneal epithelial cell sheet of the present invention causes no vascularization, unlike oral mucosal epithelial cell sheets. Thus, the corneal epithelial cell sheet of the present invention has safety superior to known cell sheets.

Further, the present invention provides induced pluripotent stem cells that can be suitably used for the induction of cell differentiation into corneal epithelial stem cells and/or corneal epithelial cells.

In addition, the corneal epithelial stem cells and/or corneal epithelial cells artificially produced by the present invention can be used as a supply of corneal epithelial stem cells and/or corneal epithelial cells that were previously obtainable only from patients. Thus, the corneal epithelial stem cells and/or corneal epithelial cells of the present invention can be a useful tool for further improvement of corneal regenerative medicine.

### Brief Description of Drawings

Fig. 1(a) shows phase contrast micrographs of iPS cell lines B31 (upper left), B34 (upper middle), B41 (upper right), C51 (lower middle), and D43 (lower left) that were established from ocular surface epithelia. For comparison, a phase contrast micrograph of human iPS cell lines 253G1 is shown (lower right).
Fig. 1(b) shows stained images of ES cell markers (ES marker) in B41 cell lines. The phase contrast micrograph (left), nuclear staining (middle), and immunostained image (right) of E-Cadherin, Nanog, Oct3/4, and Sox2 are shown.
Fig. 2(a) shows the results of karyotyic analysis of cell lines B34, B41, C51, and D43.
Fig. 2(b) shows teratoma formation by B41 cell lines (upper row), and also shows an ectoderm (a pigment epithelium and epidermis on the left-hand side in the middle row, nerves on the right-hand side in the middle row), a mesoderm (lower left), and an endoderm (lower right) that were formed in the teratoma.
Fig. 3 shows the RT-PCR measurement results on the gene expression levels of pax6 and K12 (keratin 12) in cell lines B41, 201B7, and 253G1 under differentiation induction conditions. The bottom charts show the results of analyzing the gene expression of pax6 and K12 in B41 cell lines to which BMP4 was added in the early induction stage (week 2). The horizontal axis indicates the time period, given by week, after the start of differentiation induction, and the vertical axis indicates the relative ratio of the expression level of pax6 and K12 to the expression level of the standard gene GAPDH.
Fig. 4(a) shows expression of pax6 (upper right and lower right) and K12 (upper left and lower left) in B41 cell lines under differentiation induction conditions.
Fig. 4(b) shows expression of K14 and K12 in B41 cell lines by immunostaining: phase contrast micrograph (upper left), K12 (upper right), K14 (lower left), and K14 + K12 (lower right).
Fig. 4(c) shows expression of p63, K14, and K12 in differentiation-induced 253G1 cell lines by immunostaining: phase contrast micrograph (upper left), p63 (upper right), K14 (middle left), K12 (middle right), and p63 + K12 + K14 (lower left).
Fig. 4(d) shows the results of analyzing expression of pax6, K12, and K14 in B41 cell lines that were differentiation-induced under the condition that BMP4 was added: phase contrast micrograph (upper left), K12 (upper right), pax6 (lower left), and K14 (lower right).
Fig. 4(e) shows the results of analyzing expression of pax6, K12, and K14 in 253G1 cell lines that were differentiation-induced under the condition that BMP4 was added: phase contrast micrograph (upper left), K12 (upper right), pax6 (lower left), and K14 (lower right).
Fig. 5(a) shows a phase contrast image (phase) of corneal epithelial stem cells (or progenitor cells) that were differentiation-induced from B41 cell lines, and immunostained images of pax6 and K14 (keratin 14): phase contrast micrograph (upper left), pax6 (upper right), K14 (lower left), and K14 + pax6 (lower right).
Fig. 5(b) shows a phase contrast image (phase) of corneal epithelial stem cells, and immunostained images of p63 + K14 (keratin 14): phase contrast micrograph (upper left), pax6 (upper right), K14 (lower left), and K14 + pax6 (lower right).

### Description of Embodiments

A primary object of the present invention is to provide a method for inducing differentiation of pluripotent stem cells into corneal epithelial stem cells and/or corneal epithelial cells, a method for producing corneal epithelial stem cells and/or corneal epithelial cells, a corneal epithelial cell sheet, induced pluripotent stem cells, and a cell material. The following describes the differentiation induction method, production method, corneal epithelial cell sheet, induced pluripotent stem cells, and cell material, in this order.

### 1. Differentiation Induction Method

The method of the present invention for inducing differentiation of pluripotent stem cells into corneal epithelial stem cells and/or corneal epithelial cells comprises the step of culturing pluripotent stem cells in the presence of stromal cells or an amnion-derived factor. The differentiation induction method of the present invention is described in detail below; however, this explanation is applied to the method for producing corneal epithelial stem cells and/or corneal epithelial cells, described later.

The present invention induces differentiation of pluripotent stem cells into corneal epithelial stem cells and/or corneal epithelial cells. The differentiation induction method of the present invention includes a method for inducing differentiation of pluripotent stem cells into corneal epithelial stem cells and corneal epithelial cells, a method for inducing differentiation of pluripotent stem cells into corneal epithelial stem cells, and a method for inducing differentiation of pluripotent stem cells into corneal epithelial cells. The method of the present invention is preferably, but is not limited to, a method for inducing differentiation of pluripotent stem cells into corneal epithelial stem cells.

The cornea as mentioned herein is a membrane in the form of a transparent dish that is present in the eyeballs of many mammals, including humans, and that forms the outer layer of a part in contact with the external environment, in the front of the eyeball wall. The cornea is known to be anatomically composed of, from the outside in, the corneal epithelium, corneal stroma, and corneal endothelium.

Embryologically, the corneal epithelium is derived from the ectoderm and is considered to differentiate from the epidermis. Normal corneal epithelium is known to have regenerative potential. Regeneration of the corneal epithelium is achieved by repeated self-renewal and differentiation of corneal epithelial stem cells. In other words, corneal epithelial stem cells have self-renewal ability to produce daughter cells with the same characteristics as the original, and differentiation potential to finally differentiate into corneal epithelial cells. Corneal epithelial stem cells presumably differentiate into corneal epithelial cells via corneal epithelial progenitor cells and transient amplifying cells (TA cells). Corneal epithelial stem cells in a normal cornea are present in the limbus corneae, particularly the limbal basal epithelium, which is located in the boundary of the corneal epithelium and the conjunctiva. In contrast, corneal epithelial stem cells in a cornea that develops corneal epithelial stem cell deficiency, such as Stevens-Johnson syndrome, ocular pemphigoid, or alkali injury, are irreversibly dysfunctional or afunctional.

The pluripotent stem cells to be differentiated into corneal epithelial stem cells and/or corneal epithelial cells in the present invention may be various pluripotent stem cells, as long as they have pluripotent differentiation and self-renewal ability. The pluripotent differentiation includes the ability to differentiate into ectodermal cells, preferably epidermal cells, and particularly preferably corneal epithelial cells.

The pluripotent stem cells can be suitably selected from those derived from mammals having corneal epithelial stem cells (e.g., human, mouse, monkey, cattle, dog, cat, rabbit, pig, and goat) in accordance with the purpose of use of the corneal epithelial stem cells and/or corneal epithelial cells to be differentiated. When differentiated cells are used, for example, to treat human eye diseases or to obtain development tools in the field of the treatment of human eye diseases, human-derived pluripotent stem cells are suitably used. Usable human-derived pluripotent stem cells are derived from humans of all age ranges (babies, infants, children, adolescents, young adults, middle-aged adults, and older adults), regardless of sex. When the differentiated corneal epithelial stem cells and/or corneal epithelial cells are used to treat human eye diseases, it is preferable to use pluripotent stem cells derived from an eye disease patient. When cells obtained from a patient are used, it is preferable to obtain the so-called informed consent from the patient.

Preferred specific examples of the pluripotent stem cells include, but are not limited to, embryonic stem cells and induced pluripotent stem cells.

Embryonic stem cells (ES cells) are stem cells derived from the inner cell mass of an early embryo and having self-renerewal ability and pluripotent differentiation to substantially differentiate into all cells. Usable embryonic stem cells are derived from various embryonic stem cell strains that are currently established or will be newly established in the future. In general, embryonic stem cells are known to be produced by, for example, taking an early embryo (e.g., an early embryo between fertilization and blastocyst formation) from the mother's womb, and culturing the inner cell mass of the early embryo in the presence of feeder cells (e.g., mouse embryonic fibroblasts (MEFs)); however, the technique for producing embryonic stem cells used in the present invention is not limited thereto.

Induced pluripotent stem cells (iPS cells) are cells that are derived from somatic cells and have pluripotent differentiation and self-renewal ability prepared by introducing, into somatic cells, a reprogramming factor that can induce cells into pluripotent stem cells (see, for example, PTL 1).

Induced pluripotent stem cells can be produced by various techniques that are known to a person skilled in the art or will be established in the future. More specifically, induced pluripotent stem cells can be produced by introducing, into somatic cells, a reprogramming factor that can induce cells into pluripotent stem cells. Alternatively, the induced pluripotent stem cells used in the present invention may be induced pluripotent stem cell strains that are already established.

The reprogramming factor that can induce somatic cells into pluripotent stem cells is not limited as long as it can reprogram somatic cells. Usable examples include various reprogramming factors that are currently known or will be developed in the future.

Specific examples of the reprogramming factor that can induce somatic cells into pluripotent stem cells include, but are not limited to, a combination of the OCT family, SOX family, and KLF family; a combination of the OCT family, SOX family, KLF family, and MYC family; a combination of the OCT family, SOX family, NANOG, and LIN28; and the like.

The OCT family is, for example, at least one member selected from the group consisting of OCT3/4, OCT1A, and OCT6, but is not limited thereto. The SOX family is, for example, at least one member selected from the group consisting of SOX1, SOX2, SOX3, SOX7, SOX15, SOX17, and SOX18, preferably SOX1 and/or SOX2, but is not limited thereto. The KLF4 is, for example, one or more members selected from the group consisting of KLF1, KLF2, KLF4, and KLF5, preferably at least one member selected from the group consisting of KLF2, KLF4, and KLF5, but is not limited thereto. The MYC family is, for example, at least one member selected from the group consisting of C-MYC, N-MYC, and L-MYC, but is not limited thereto.

Preferred examples of the reprogramming factor that can induce somatic cells into pluripotent stem cells include widely known reprogramming factors, including a) human reprogramming factors, such as a combination of the three factors of OCT3/4, SOX2, and KLF4; a combination of these three factors and additionally C-MYC; and a combination of the four factors of OCT3/4, SOX2, NANOG, and LIN28; and including b) mouse reprogramming factors, such as a combination of the three factors of Oct3/4, Sox2, and Klf4; and a combination of these three factors and additionally c-Myc. OCT3/4, SOX2, KLF4, and C-MYC (Oct3/4, Sox2, Klf4, and c-Myc) are collectively known as the "Yamanaka factors."

The reprogramming factor that can induce somatic cells into pluripotent stem cells may be either genes (nucleic acid molecules) or gene products (proteins), as long as they can induce somatic cells into pluripotent stem cells; however, in terms of increasing the efficiency of establishing induced pluripotent stem cells, genes (nucleic acid molecules) are preferably used. The base sequences (genes) and amino acid sequences (proteins) of the specific inducing factors mentioned above are both known to a person skilled in the art. These base sequences and amino acid sequences are available in, for example, the database published by the National Center for Biotechnology Information (NCBI).

The reprogramming factor that can induce somatic cells into pluripotent stem cells, and the somatic cells into which the reprogramming factor is to be introduced, may be derived from organisms of the same or different species. In terms of increasing the efficiency of establishing induced pluripotent stem cells, the reprogramming factor and the somatic cells are preferably derived from organisms of the same species.

The method for introducing the reprogramming factor that can induce somatic cells into pluripotent stem cells, into the somatic cells to be reprogrammed can be suitably selected by a person skilled in the art. For example, when a gene is used as the reprogramming factor, the reprogramming factor can be introduced into somatic cells by a method generally used in transfection of animal cells. Specific examples of the method for introducing the reprogramming factor into somatic cells include a method using a vector, a calcium phosphate method, a lipofection method, an electroporation method, a microinjection method, and the like. Among these, a method using a vector is preferred in terms of induction efficiency. Vectors that can be used to introduce the reprogramming factor into somatic cells are, for example, viral vectors, non-viral vectors, and artificial viruses. In terms of safety, viral vectors, such as lentivirus, adenovirus, and retrovirus, are suitably used. When vectors are used, the reprogramming factor genes may be each incorporated into different vectors, or two or more genes may be incorporated into one vector.

The induced pluripotent stem cells may be derived from various somatic cells, as long as induced pluripotent stem cells can be induced. The induced pluripotent stem cells are preferably induced from normal somatic cells, rather than tumor somatic cells.

The cells used to obtain the induced pluripotent stem cells are not limited, and include any cells for which the production of iPS cells has been confirmed (e.g., fibroblasts) or will be reported in the future. In terms of particularly high efficiency of inducing differentiation into corneal epithelial cells, it is preferable to use induced pluripotent stem cells obtained by introducing the reprogramming factor that can induce cells into pluripotent stem cells, into cells derived from the ocular surface epithelium. The ocular surface epithelium-derived cells can be obtained from, for example, but are not limited to, a tissue sample of about 2 mm² harvested from corneal limbal epithelium, corneal epithelium, or conjunctival epithelium. The ocular surface epithelium-derived cells may be cells obtained from tissue samples harvested in the above manner or cells obtained by suitably culturing such cells.

The somatic cells, into which the reprogramming factor that can induce cells into pluripotent stem cells has been introduced, are induced into pluripotent stem cells by culturing. A person skilled in the art can suitably select the medium used, culture conditions, and culture period, from a wide range.

When induced pluripotent stem cells are produced, the production of induced pluripotent stem cells can be confirmed by a technique known to a person skilled in the art. For example, the production of induced pluripotent stem cells can be readily confirmed by measuring the expression of embryonic stem cell markers, such as SSEA3, SSEA4, E-Cadherin, Nanog, Oct3/4, and Sox2, but the technique is not limited thereto.

In the culture step, pluripotent stem cells are cultured in the presence of stromal cells or an amnion-derived factor.

When the culture of pluripotent somatic cells is performed in the presence of stromal cells, the specific means can be suitably selected by a person skilled in the art. For example, the culture in the presence of stromal cells is performed using the stromal cells as feeder cells. More specifically, the culture in the presence of stromal cells can be performed by culturing pluripotent somatic cells while the stromal cells reach a confluent state in the culture vessel. The "confluent state" as mentioned herein, which is known to a person skilled in the art, means that the cells are grown to the number of cells covering almost the entire surface of the culture vessel. When used as feeder cells, the stromal cells may be subjected to mitomycin-C (MMC) treatment, X-ray irradiation, or the like, to inactivate cell division, if necessary.

The stromal cells can be suitably selected by a person skilled in the art within a range that can achieve the object of the present invention. Stromal cells generally refer to sustentacular cells that surround tissues (e.g., epithelium and endothelium) or organs. Preferably usable stromal cells include, but are not limited to, those used by a person skilled in the art in a technique known as the SDIA method (PTL 2 and NPL 3).

Specific examples of the stromal cells include, but are not limited to, mouse calvaria-derived MC3T3-G2-/PA6 cells, M-CSF deficient mouse calvaria-derived OP9 cells, mouse fetus-derived NIH/3T3 cells, and the like. These stromal cells can be used singly or in combination of two or more. Preferable stromal cells are mouse calvaria-derived MC3T3-G2-/PA6 cells. The above cells are all known to, and easily available to, a person skilled in the art.

The SDIA method as mentioned herein is a method of inducing differentiation of embryonic stem cells into ectodermal cells (neural stem cells, nerve cells, neural crest cells, or neural tubes), and is a known method, as shown in PTL 2 and NPL 3. The characteristic of the SDIA method is the differentiation induction of embryonic stem cells in the presence of stromal cells. The method is considered to be a technique of suppressing the differentiation of embryonic stem cells into epidermal cells or the like, thereby inducing their differentiation into nerve cells, etc.

The culture in the presence of an amnion-derived factor can be performed by the method disclosed in PTL 4. The culture in the presence of an amnion-derived factor can be achieved by, but is not limited to, culturing the cells in the presence of an amnion or a processed product derived from an amnion. The amnion or the amnion-derived processed product may be taken from an organism that is the same or different from the organism from which the pluripotent stem cells to be cocultured are derived. The amnion or the amnion-derived processed product can be suitably obtained by a person skilled in the art based on a known technique.

The culture is performed in the presence of stromal cells or an amnion-derived factor. The "culture in the presence of stromal cells or an amnion-derived factor" is not limited, as long as the pluripotent stem cells are cultured in a state in which material exchange can occur between the pluripotent stem cells and the stromal cells or the amnion-derived factor. More specifically, when the culture is performed in the presence of stromal cells, the stromal cells and the pluripotent stem cells may be in a contact or non-contact state.

The state of the pluripotent stem cells is not limited, as long as the culture is performed in the presence of stromal cells or an amnion-derived factor. The pluripotent stem cells may be in the form of clumps or single cells. When the pluripotent stem cells are induced pluripotent stem cells or embryonic stem cells, it is preferable to, but not limited to, culture the stem cells in the form of clumps.

The term "clumps" as mentioned herein indicates that the culture is started while cell-cell adhesions (clumps) are present, rather than the pluripotent stem cells to be cultured being completely dispersed by enzyme treatment, etc., and that the culture is continued while cell-cell adhesions are present. Means for aggregating the cells into clumps are known to a person skilled in the art, and a person skilled in the art can suitably select one from the known means. Generally, a clump of pluripotent stem cells contains, but is not limited to, about 50 to 10,000 pluripotent stem cells. The clumps contain embryoid bodies.

In the culture, the abundance of the pluripotent stem cells relative to the stromal cells or amnion-derived factor is not limited, and can be suitably determined by a person skilled in the art. When the culture is performed in the presence of stromal cells, the abundance of the pluripotent stem cells relative to the stromal cells is preferably about 1 to 150 clumps, and more preferably about 15 to 40 clumps, based on 3.8 cm² of a confluent stromal cell layer. "3.8 cm²" refers to the area per well of a 12-well plate generally used as a culture vessel.

The culture is performed over a period that allows the differentiation of the cells into corneal epithelial stem cells and/or corneal epithelial cells to be achieved. The culture period may vary depending on factors such as the type of pluripotent stem cells used, can be suitably determined by a person skilled in the art, and is not limited. For example, the culture can be continued until the expression of specific marker genes is observed in corneal epithelial stem cells and/or corneal epithelial cells, described later. In terms of achieving sufficient differentiation induction, the culture is preferably performed, for example, for about 6 weeks or more, preferably about 8 weeks or more, and particularly preferably about 12 weeks or more. For example, the culture period may be about 6 to 20 weeks, preferably about 8 to 16 weeks, and more preferably about 8 to 14 weeks.

A serum-free medium is preferably used in the culture step of the present invention. A person skilled in the art can suitably select the serum-free medium from media that do not substantially contain serum, can culture mammalian cells, and are known or will be developed in the future. Examples of the serum-free medium include, but are not limited to, Glasgow minimum essential medium (G-MEM), Dulbecco's modified Eagle's medium (D-MEM), and the like. The serum-free medium may contain additives, such as about 5 to 20%, preferably about 10%, knockout serum replacement (trade name: KSR, produced by Invitrogen); about 0.5 to 5 mM, preferably about 2 mM, L-glutamine; about 0.5 to 5%, preferably about 1%, sodium pyruvate (Gibco); about 0.5 to 5%, preferably about 1%, nonessential amino acids (Gibco); and about 0 to 1%, preferably about 0.1%, 2-mercaptoethanol.

It is preferable that the serum-free medium is regularly exchanged in a standard manner. A person skilled in the art can exchange the serum-free medium by a known technique. When the serum-free medium is exchanged, the serum-free media used before and after the exchange may have the same composition or different compositions. A person skilled in the art can appropriately set the time between exchanges and the frequency of exchange. The medium is preferably exchanged once every about one to three days, and more preferably once every about two days. When the stromal cells are used as feeder cells, the feeder cells are generally not exchanged; however, it is not limited thereto.

It is preferable that the serum-free medium does not substantially contain BMP4 for at least about one to two weeks (e.g., about two weeks) after initiation of culture. BMP4 (bone morphogenetic protein 4) is a known protein. Prior to the present invention, BMP4 was considered to have the function of suppressing neuronal differentiation of ectodermal cells, and inducing differentiation of the cells into epithelial cells (e.g., epidermis and corneal epithelium). It is particularly preferable that the serum-free medium does not substantially contain BMP4 for at least about one to two weeks after the start of the culture step. The expression that the serum-free medium "does not substantially contain BMP4" as mentioned herein means that the BMP4 content of the medium may be less than an amount that does not affect the effect of the differentiation induction method of the present invention. For example, the BMP4 content is preferably less than 0.5 nM, more preferably less than 0.1 nM, and even more preferably less than 0.05 nM. From another viewpoint, it is preferable that the medium does not intentionally contain BMP4. A commercially available medium reagent sold as a medium that does not contain BMP4 and a combination of such medium reagents can be readily used as the serum-free medium that does not substantially contain BMP4.

The serum-free medium may be one that does not substantially contain retinoic acid, but is not limited thereto.

The culture can be performed by a person skilled in the art by a known technique using, for example, a serum-free medium mentioned above. A preferred example of the technique of performing the culture is, but is not limited to, a technique of performing the culture at about 37°C at a carbon dioxide concentration of about 5%. The culture under such conditions can be performed by using, for example, a known CO₂ incubator.

The method for inducing differentiation of cells into corneal epithelial stem cells and/or corneal epithelial cells is thus achieved. The corneal epithelial stem cells differentiated by the method of the present invention may contain corneal epithelial precursor cells.

The differentiation into corneal epithelial stem cells and/or corneal epithelial cells can be confirmed by a means known to a person skilled in the art. As a specific technique for confirming the differentiation, for example, some of the cells during culture are taken regularly (e.g., every two weeks), and the expression of a marker gene (e.g., pax6 gene specifically expressed in ocular tissue, or K12 (keratin 12) gene specifically expressed in differentiated corneal epithelium) is detected by quantitative RT-PCR assay. In this case, the confirmation of the expression of the marker gene can be used as an indicator of the differentiation of the cells into corneal epithelial stem cells and/or corneal epithelial cells.

### 2. Production Method

The method for producing corneal epithelial stem cells and/or corneal epithelial cells of the present invention comprises the following steps: (1) culturing pluripotent stem cells, and (2) selecting corneal epithelial stem cells and/or corneal epithelial cells from among the cultured cells.

The production method of the present invention is described below in detail.

Step (1) of culturing pluripotent stem cells is equivalent to the step of culturing pluripotent stem cells described in "1. Differentiation Induction Method" above. Specifically, step (1) is for culturing pluripotent stem cells in the presence of stromal cells or an amnion-derived factor. Step (1) may be continued, for example, until a corneal epithelial stem cell-specific marker and/or a corneal epithelial cell-specific marker is expressed in the cultured pluripotent stem cells.

Subsequently, in step (2), corneal epithelial stem cells and/or corneal epithelial cells are selected from among the cells (population) cultured in step (1). Means for selecting corneal epithelial stem cells and/or corneal epithelial cells may be appropriately determined by a person skilled in the art.

The following is an example of one embodiment of a means for selecting corneal epithelial stem cells. Specifically, corneal epithelial stem cells are selected from among the cells (population) cultured in step (1) by using self-renewal ability and/or expression of a corneal epithelial stem cell-specific marker as an index. More specifically, corneal epithelial stem cells can be selected through a step of selecting cells having self-renewal ability and a step of selecting cells expressing a corneal epithelial stem cell-specific marker, although the selecting means is not limited thereto. The order of the step of selecting cells having self-renewal ability and the step of selecting cells expressing a corneal epithelial stem cell-specific marker is not limited. Whichever step comes first, corneal epithelial stem cells can be selected.

The renewal ability of the cells can be evaluated by a technique known to a person skilled in the art. For example, it can be evaluated by inoculating cells into a culture system for subculture, and confirming the renewal of the cells, although the technique for evaluation is not limited thereto. The renewal of cells can be confirmed by the formation of colonies on subculture. A co-culture system with feeder cells (e.g., NIH/3T3 cells) or a medium for epithelial stem cells is preferably used for the subculture for confirming colony formation, although it is not limited thereto.

The medium for subculture is not particularly limited, as long as corneal epithelial stem cells can be cultured therein (e.g., keratinocyte-conditioned medium (KCM), keratinocyte serum-free medium (KSFM, available from Invitrogen, etc.), CnT-20 medium (CELLnTEC), and CnT-50 medium (CELLnTEC), all of which are known to a person skilled in the art).

When, for example, a 12-well plate is used for the subculture, the cells are preferably reinoculated at a density of about 2,000 to 16,000 cells/well.

The renewal ability is preferably self-replication ability. The self-renewal ability of cells is evaluated according to, for example, the indices, i.e., whether the characteristics of the renewed cells are unchanged due to subculture, in addition to whether the cells have renewal ability. The evaluation is not, however, limited thereto. The characteristics of renewed cells can be suitably confirmed with the use of corneal epithelial stem cell markers described below, although how to confirm the cell characteristics is not limited thereto.

Expression of a corneal epithelial stem cell-specific marker in the cells can be evaluated by a technique known to a person skilled in the art. Examples of corneal epithelial stem cell markers include, but are not limited to, K14 (keratin 14) protein and/or p63 protein and/or N-cadherin protein, and the like, which are layered epithelium stem cell-specific (and progenitor cell-specific) markers. Additionally, ocular tissue-specific markers (e.g., pax6 protein) and/or corneal epithelial-specific differentiation markers (e.g., K12 (keratin 12) protein) can also be detected. After detection of corneal epithelial-specific differentiation markers (K12), it is also possible to detect cell surface markers (e.g., Integrin alpha 6, N-cadherin) expressed specifically in corneal epithelial stem cells.

There are known specific methods for detecting expression of corneal epithelial stem cell markers; however, detection by immunocytochemistry, for example, is suitable. Specific examples include detection by means of microscope observation using antibody staining, detection of cell surface markers by a cell sorter (flow cytometer), and the like. It is also possible to perform detection by introducing a reporter gene of the corneal epithelial stem cell marker (e.g., a reporter gene in which a promoter region of a gene encoding the corneal epithelial stem cell marker is associated with a fluorescent protein such as green fluorescent protein (GFP)) into pluripotent stem cells that are to be induced for differentiation, and, after differentiation induction, detecting expression of the reporter.

To select corneal epithelial cells, for example, a step of selecting cells expressing a corneal epithelial cell marker may be performed. Examples of corneal epithelial cell markers include, but are not limited to, ocular tissue-specific markers (e.g., pax6 protein) and/or corneal epithelial-specific differentiation markers (e.g., K12 (keratin 12) protein). Examples of means for detecting expression of a corneal epithelial cell marker include detection performed by introducing a reporter gene of the corneal epithelial cell marker (e.g., a reporter gene in which a promoter region of a gene encoding the corneal epithelial cell marker is associated with a fluorescent protein such as green fluorescent protein (GFP)) into pluripotent stem cells that are to be induced for differentiation, and, after differentiation induction, detecting expression of the reporter.

To select corneal epithelial stem cells, for example, (a) and (b) below are performed as a specific embodiment of step (2) above, but are not limited thereto: (a) after induction of cell differentiation in step (1), the cells expressing a corneal epithelial stem cell marker are confirmed, the cells are then recovered by enzymatic treatment or the like and reinoculated into a system that can selectively culture corneal epithelial stem cells (e.g., a co-culture system using NIH/3T3 cells as feeder cells), and renewed cells are selected using the colony formation as an index to thereby purify corneal epithelial stem cells; and (b) after induction of cell differentiation in step (1), the cells expressing a corneal epithelial-specific marker are confirmed, and the cells expressing a corneal epithelial stem cell-specific cell surface marker are selected by using a cell sorter or the like, to thereby purify corneal epithelial stem cells.

In this manner, corneal epithelial stem cells and/or corneal epithelial cells are produced. The corneal epithelial stem cells obtained through the production method of the present invention may include corneal epithelial progenitor cells.

### 3. Corneal Epithelial Cell Sheet

The corneal epithelial cell sheet of the present invention is obtained from the corneal epithelial stem cells and/or the corneal epithelial cells obtained by the method described in "2. Production Method" above. The corneal epithelial cell sheet may be layered.

The means for producing a corneal epithelial cell sheet may be suitably selected from the techniques known to a person skilled in the art or techniques that will be established in the future. For example, the following methods disclosed in PTL 3 or PTL 4, or NPL 1 or NPL 2, may be used.

Specific examples of means for producing a corneal epithelial cell sheet include, but are not particularly limited to, a means comprising culturing the corneal epithelial stem cells on a temperature-responsive culture dish or a carrier, in the presence of feeder cells, and recovering the cell sheet obtained in the previous step.

As the feeder cells used in the culture step above, NIH/3T3 cells treated with mitomycin C (MMC) may be used, although the feeder cells are not limited thereto.

The temperature-responsive culture dish used in the culture step is not particularly limited, as long as it is a culture dish from which a cell sheet can be peeled off when the temperature changes (preferably when the temperature decreases). Specific examples include a culture dish covered with a temperature-responsive polymer such as poly(N-isopropylacrylamide). For convenience, a commercially available temperature-responsive culture dish can also be used.

Examples of the carriers include, but are not limited to, extracellular matrices, such as collagen, laminin, and fibronectin (preferably collagen).

The conditions for the culture step can be suitably determined by a person skilled in the art. For example, the culture may be performed at about 37°C at a CO₂ concentration of about 5%, although the conditions are not limited thereto. The culture period may be set to about two weeks.

When the culture is carried out on a temperature-responsive culture dish, the recovery can be performed by decreasing the temperature and peeling the formed cell sheet off from the temperature-responsive culture dish. When the culture is carried out on a carrier, the formed sheet can be recovered together with the carrier.

The corneal epithelial cell sheet can be suitably used, for example, for the treatment of refractory eye diseases, such as Stevens-Johnson syndrome, ocular pemphigoid, and alkali injury.

### 4. Induced Pluripotent Stem Cells That Are Efficiently Differentiated into Corneal Epithelial Stem Cells and Corneal Epithelial Cells

Induced pluripotent stem cells that are obtained by introducing a reprogramming factor, which can induce cells into pluripotent stem cells, into ocular surface epithelium-derived cells are useful, since they can be more promptly and more efficiently induced to differentiate into corneal epithelial stem cells and/or corneal epithelial cells, compared to induced pluripotent stem cells that are obtained by introducing a reprogramming factor into fibroblasts or the like.

The ocular surface epithelium-derived cells described in "1. Differentiation induction Method" above are used as the ocular surface epithelium-derived cells mentioned above. Specific examples include, but are not limited to, ocular surface epithelium-derived cells that can be obtained from a tissue sample of about 2 mm² harvested from corneal limbal epithelium, corneal epithelium, or conjunctival epithelium. The ocular surface epithelium-derived cells may be obtained from a tissue sample harvested as above, or obtained by suitably culturing these cells.

The reprogramming factors, which can induce cells into pluripotent stem cells, described in "1. Differentiation Induction Method" are suitably used as the reprogramming factor mentioned above, which can induce cells into pluripotent stem cells. The means for introducing the factor into the cells described in "1. Differentiation Induction Method" above is also suitably used as the means for introducing the reprogramming factor, which can induce cells into pluripotent stem cells, into ocular surface epithelium-derived cells.

The induced pluripotent stem cells of the present invention have a high propensity to be induced to differentiate into corneal epithelial stem cells, as described in the Examples below. Here, for example, the propensity of a corneal epithelial-specific marker to express a keratin 12 (K12) can be used as the "propensity to be induced to differentiate into corneal epithelial stem cells." When the propensity to express K12 serves as the high propensity to be induced to differentiate into corneal epithelial stem cells, K12 is expressed at high levels in an early stage after induction of cell differentiation is initiated. For this reason, the induced pluripotent stem cells of the present invention can be suitably used for induction of cell differentiation into corneal epithelial stem cells.

### 5. Cell Material

As described above, the induced pluripotent stem cells of the present invention have a high propensity to be induced to differentiate into corneal epithelial stem cells. The present invention therefore also provides a cell material used for induction of cell differentiation into corneal epithelial stem cells, including induced pluripotent stem cells described in "4. Induced Pluripotent Stem Cell" above.

The cell material can be suitably used for induction of cell differentiation into corneal epithelial stem cells and for production of corneal epithelial stem cells. Further, the obtained corneal epithelial cells can be used, for example, in the production of a corneal epithelial cell sheet, as a drug discovery tool, but applications are not limited to these examples.

### Example

Hereinafter, the present invention will be described in detail with reference to the Example. However, the scope of the present invention is not limited thereto.

### I: Generation of iPS Cells Originated from Ocular Surface Epithelial Cells

From imported sclerocorneal pieces for use in research, human-derived corneal endothelial cells, peripheral connective tissues, etc., were removed, and only the corneal-conjunctival epithelial layers on the ocular surfaces were isolated by dispase treatment. The obtained epithelial cells were dissociated into single cells by trypsin treatment and cultured in a medium containing a culture supernatant of NIH/3T3 cells and a fresh KCM in a 1:1 ratio.

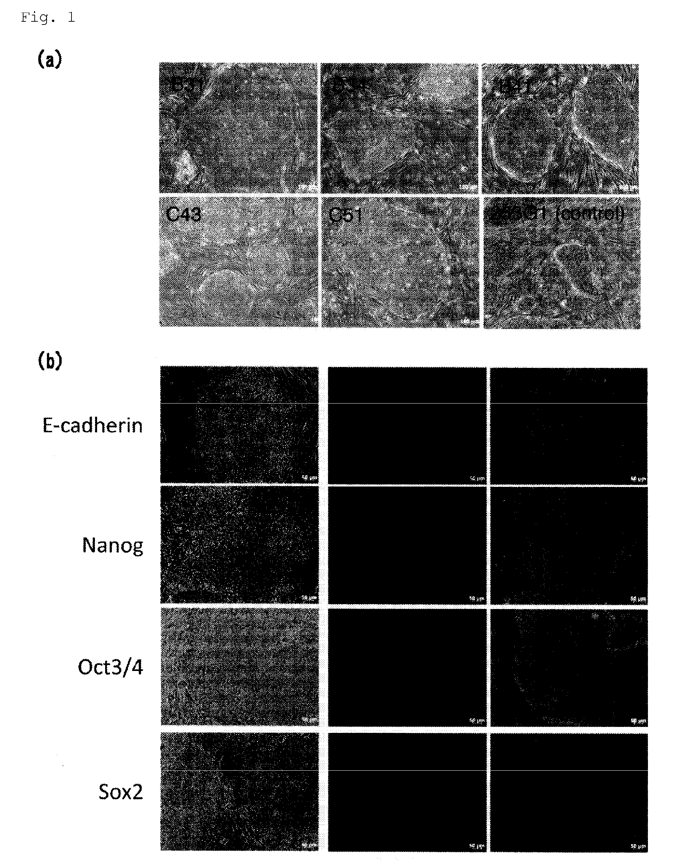

Subsequently, the proliferated epithelial cells orginated from ocular surfaces were transfected with lentiviral vectors into each of which, human Oct3/4, Sox2, Klf4, and c-Myc (Yamanaka 4 factors) were individually introduced. Two to four days after the transfection, the resulting cells were re-inoculated into an iPS cell culture system (i.e., re-inoculated on MEF feeder cells in an iPS cell culture), and cultured for about 3 to 6 weeks. After confirming the absence of the exogenous Yamanaka 4 factors, SSE4 antibody-positive colonies were selected, thereby establishing iPS cell lines (B31, B34, B41, C51, and D43; 5 types of cell lines in total). Fig. 1(a) shows phase contrast micrographs of the established iPS cell lines and previously reported iPS cell line, 253G1. These established human iPS cell lines all formed colonies with cobblestone morphology, which is characteristic of pluripotent stem cells, on the MEF feeder cells, as seen in known human-fibroblast-derived iPS cell lines.

One type of the generated iPS cell lines, B41 cell lines, was examined for expression of ES cell markers, i.e., E-cadherin (H-108 rabbit polyclonal antibody manufactured by SantaCruz, 50-fold dilution), Nanog (goat polyclonal antibody manufactured by R&D, 100-fold dilution), Oct3/4 (goat polyclonal antibody manufactured by R&D, 100-fold dilution), and Sox2 (245610 mouse monoclonal antibody manufactured by R&D, 100-fold dilution) by immunostaining. For a secondary antibody, Alexa568 antibody (manufactured by Invitrogen, 200-fold dilution) directed against each animal species of the primary antibodies was used. Lastly, the nuclei were stained with Hoechst 33342. Fig. 1(b) shows the immunostained images. It was seen that the generated iPS cells expressed all 4 types of ES cell markers, i.e., E-cadherin, Nanog, Oct3/4, and Sox2. In other words, iPS cells were considered to have been normally induced.

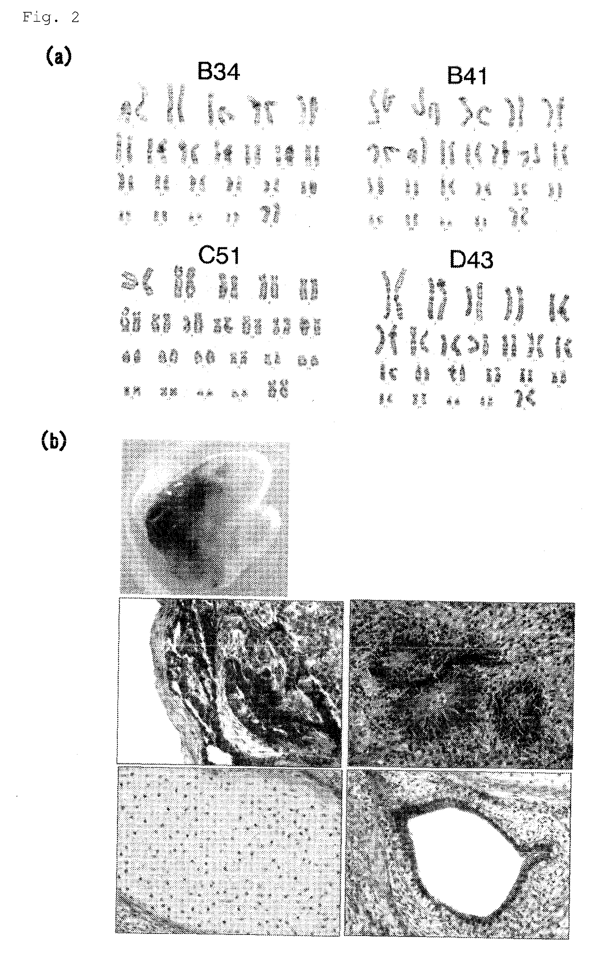

The generated 4 types of iPS cell lines (B34, B41, C51, and D43) were examined for karyotype in accordance with an ordinary method. Fig. 2(a) shows the results of karyotype analysis. In 50 cells out of 50 for each of the examined 4 types of human iPS cell lines, a karyotype of 46, XX[20], which is a normal human karyotype, was observed.

Furthermore, the obtained iPS cells were tested in a teratoma formation assay in accordance with an ordinary method. Fig. 2(b) shows the results. In teratomas, an ectoderm (retinal pigment epithelium, epidermis, and nerves), a mesoderm, and an endoderm were observed.

### II: Differentiation Induction into Corneal Epithelial Cells and Corneal Epithelial Stem Cells

The iPS cells (B41 cell lines) generated in section I above, and known human-fibroblast-derived cell lines 253G1 and 201B7, were cultured on a feeder layer of MC3T3-G2/PA6 cells (PA6 cells).
(1) The three types of human iPS cells were individually subcultured on mouse embryonic fibroblast (MEF) feeder cells in the medium described below.
   Medium for Human iPS Cells
   DMEM/F12 (Invitrogen)
   25% KSR (Invitrogen)
   2 mM L-Glutamine (Invitrogen)
   1% nonessential amino acids (Invitrogen, 100x)
   0.1% 2-mercaptoethanol (Invitrogen, 1,000x)

After culturing, a dissociation solution was added to each type of the human iPS cells to dissociate them into clumps (cell clumps), followed by collection of the clumps.

(2) The collected clumps were inoculated in a culture dish coated with a 0.1% gelatin solution, and incubated at 37°C for 45 minutes to thereby remove the MEF feeder cells. This is performed probably because the MEF feeder cells adhere to the culture dish first, and the human iPS cells therefore adhere less, thereby remaining in a supernatant.
(3) The human iPS cell clumps were collected and inoculated in such a manner as to have a density of 20 to 40 clumps per well in a 12-well plate in which mouse calvaria-derived MC3T3-G2/PA6 cells (PA6 cells) were inoculated as a feeder layer. A medium of the following composition was used. Each type of the iPS cells was cultured at a temperature of 37°C with a carbon dioxide concentration of 5%.

### Cornea Induction Medium

G-MEM (Invitrogen)
10% KSR (Invitrogen)
2mM L-Glutamine (Invitrogen)
1% Pyruvate (Invitrogen, 100x)
1% nonessential amino acids (Invitrogen, 100x)
0.1% 2-mercaptoethanol (Invitrogen, 1,000x)

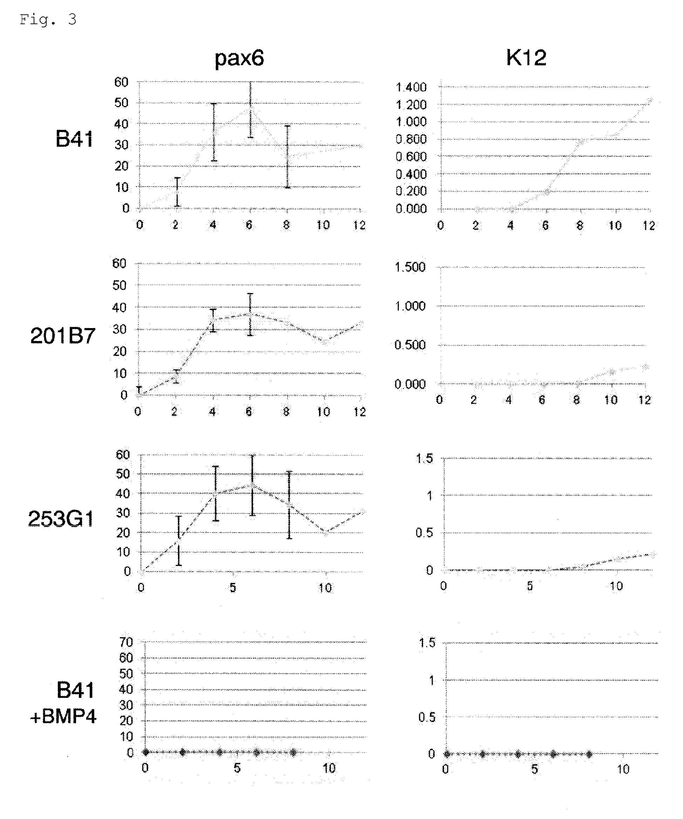

(4) Differentiation induction was performed for 12 to 15 weeks. The cells were collected every two weeks, and expression of an ocular-tissue-specific marker pax6 and a corneal-epithelium-specific marker K12 was analyzed by real-time PCR. Further, B41 cell lines were also cultured under the same conditions except that BMP4 was added to the medium in such a manner as to make a final concentration of 0.5 nM, and expression of pax6 and K12 was analyzed by real-time PCR. Fig. 3 shows the results.

In B41 cell lines, expression of the ocular-tissue-specific marker pax6 was induced. Furthermore, 6 to 8 weeks after the induction, an increase in expression of K12, a mature corneal-epithelium-specific marker, was observed. In contrast, while expression of the ocular-tissue-specific marker pax6 was observed in fibroblast-derived cell lines 201B7 and 253G1 as much as seen in B41 cell lines, expression of K12 was observed in the eighth week, which was 4 weeks later than B41 cell lines. When BMP4 was added to B41 cell lines in the early stage of induction (week 2), expression of neither pax6 nor K12 was observed.

The expression level given by real-time PCR was calculated as a relative expression level compared to that of a standard gene GAPDH. To detect the expression level of each gene, commercially available TaqMan Gene Expression Assays (manufactured by Applied Biosystems) were used as probes.
The probes used:
TaqMan Probe
Pax6: Hs00240871_m1
K12: Hs00165015_m1
GAPDH: Hs99999905_m1

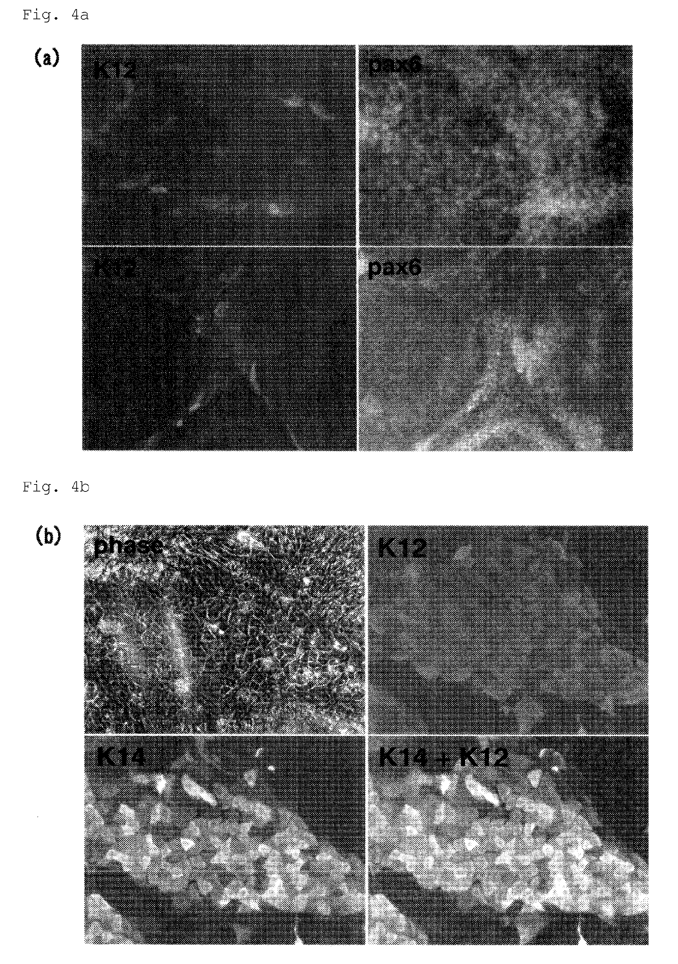

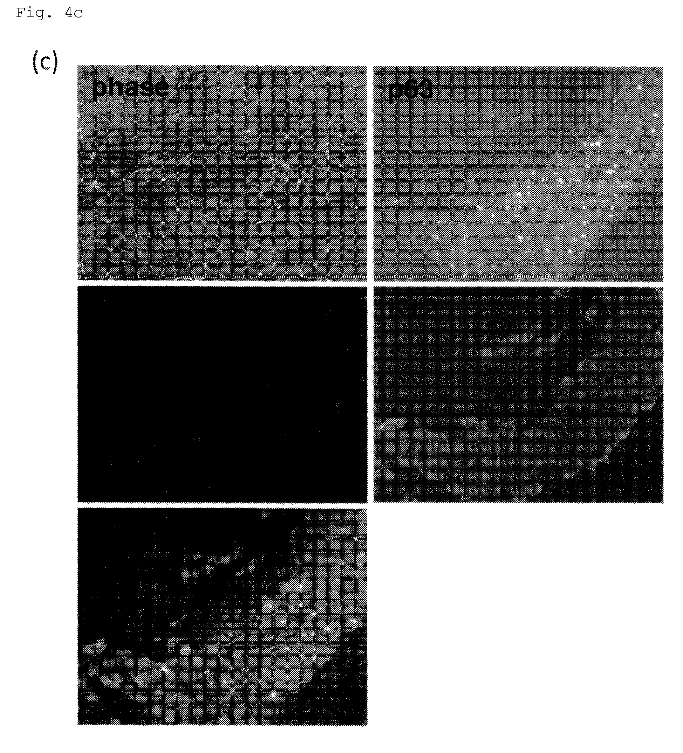

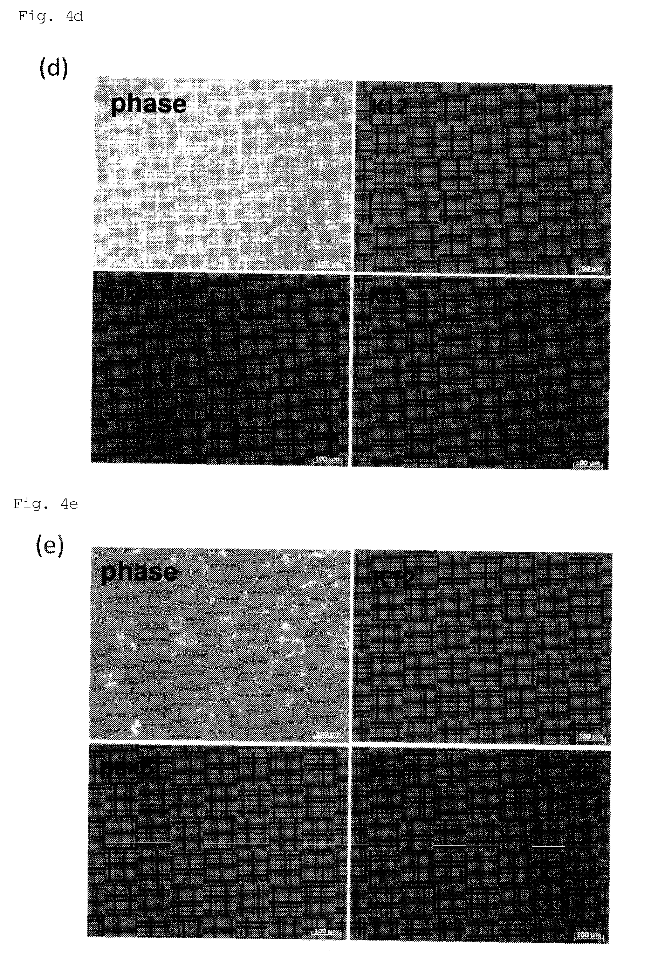

(5) The cells derived from differentiation-induced B41 cell lines were fixed, and colocalization of pax6 and K12, and colocalization of K14 and K12, were confirmed by immunostaining. Fig. 4 shows the stained images. In some of the colonies present after the differentiation induction, cell populations that were positive for the corneal-epithelium-specific marker K12 and were also positive for the ocular-tissue-specific marker pax6 were observed (Fig. 4(a)). Further, it became evident that an epithelial progenitor cell marker K14 and the corneal-epithelium-specific marker K12 colocalized in some cells, while they were individually present in other cells (Fig. 4(b)). These expression patterns are the same as those of corneal epithelia and limbal epithelia in vivo.

After a prolonged period of differentiation induction (about 10 weeks or more), fibroblast-derived 253G1 cell lines were examined for expression and colocalization of p63, K12, and K14 in the same manner as B41 cell lines. As a result, p63, K12, and K14 were found colocalized in some cells, while being found individually present in other cells (Fig. 4c). On the other hand, neither B41 cell lines nor 253G1 cell lines, to both of which BMP4 was added in the early culture stage to perform differentiation induction, expressed K12, K14, or pax6 (Figs. 4(d) and 4(e)). This indicates that addition of BMP4 in an amount of 0.5 nM inhibited B41 cell lines and 253G1 cell lines from being induced to differentiate into corneal epithelia.

(6) In the eighth to twelfth week of the differentiation induction, iPS cells (B41 cell lines) were collected by trypsin treatment, and inoculated into a co-culture system (epithelial stem cell culture system) containing NIH/3T3 cells, which served as feeder cells. A re-inoculation density of about 8,000 cells per well (12-well plate) was established. After re-inoculation, the following medium was used.

### KCM Medium Composition

69% Dulbecco's Modified Eagle's Medium (DMEM) (Sigma-Aldrich)
23% Nutrient Mixture F-12 Ham (Sigma-Aldrich)
5% Fetus Bovine Serum (FBS) (Japan Bio Serum)
1% Penicillin-Streptomycin (Invitrogen, 100x)
0.4 µg/ml Hydrocortisone Succinate (Wako)
2 nM 3,3',5-Triiodo-L-thyronine sodium salt (MP Biomedicals)
100 nM Cholera Toxin (Calbiochem)
2 mM L-Glutamine (Invitrogen)
0.5% Insulin Transferrin Selenium (Gibco, 200x)
10 ng/ml Recombinant Human EGF (R&D Systems)

After culturing for 2 to 3 weeks, the resulting cells were fixed, and expression of pax6, K12, p63, and K14 was confirmed by immunostaining.

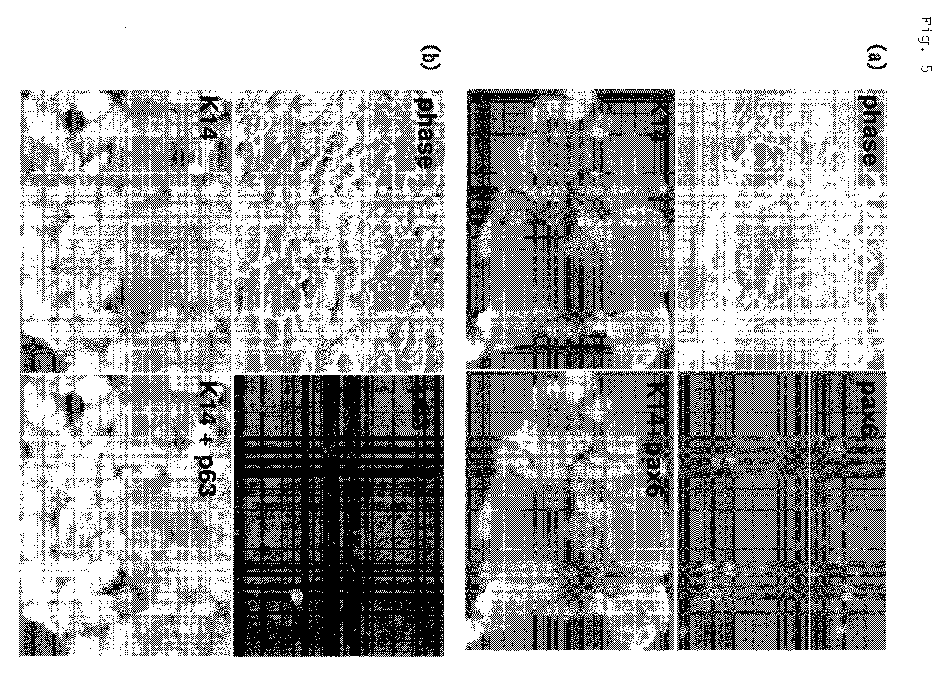

Fig. 5 shows the phase contrast images and immunostained images. The obtained colonies derived from B41 cell lines exhibited cobblestone morphology, which is unique to epithelial cells, and exhibited expression of the corneal epithelial progenitor cell marker K14 and pax6 (Fig. 5(a)). Furthermore, expression of p63, which is also a corneal epithelial progenitor cell marker, and K14 was also observed (Fig. 5(b)).

## Claims

1. A method for inducing differentiation of a pluripotent stem cell into a corneal epithelial stem cell and/or a corneal epithelial cell,
the method comprising the step of culturing a pluripotent stem cell in the presence of a stromal cell or an amnion-derived factor.

2. The method according to claim 1, wherein the pluripotent stem cell is cultured in a serum-free medium.

3. The method according to claim 2, wherein the serum-free medium substantially does not contain BMP4 at least during the period from the beginning of the culture to 1 to 2 weeks after the culture.

4. The method according to any one of claims 1 to 3, wherein the pluripotent stem cell is an embryonic stem cell (ES cell) or an induced pluripotent stem cell (iPS cell).

5. The method according to claim 4, wherein the induced pluripotent stem cell is obtained by introducing a reprogramming factor, which can induce a cell into a pluripotent stem cell, into an ocular surface epithelium-derived cell.

6. A method for producing a corneal epithelial stem cell and/or a corneal epithelial cell, comprising the step of:
(1) culturing a pluripotent stem cell in the presence of a stromal cell or an amnion-derived factor; and
(2) selecting a corneal epithelial stem cell and/or a corneal epithelial cell from among the cultured cells.

7. The method according to claim 6, wherein the step of selecting a corneal epithelial stem cell and/or a corneal epithelial cell from among the cultured cells is a step of selecting a cell that has self-renewal ability and that expresses a corneal epithelial stem cell-specific marker.

8. A corneal epithelial cell sheet obtained from the corneal epithelial stem cell and/or the corneal epithelial cell obtained by the method according to claim 6 or 7.

9. An induced pluripotent stem cell obtained by introducing a reprogramming factor, which can induce a cell into a pluripotent stem cell, into an ocular surface epithelium-derived cell.

10. The induced pluripotent stem cell according to claim 9, wherein the induced pluripotent stem cell is used for differentiation induction into a corneal epithelial stem cell and/or a corneal epithelial cell.

11. A cell material for differentiation induction into a corneal epithelial stem cell and/or a corneal epithelial cell, the cell material comprising the induced pluripotent stem cell according to claim 9 or 10.
